# EUROPEAN PATENT APPLICATION

(11) **EP 2 015 075 A1**
(43) Date of publication of application: **14.01.2009**
(21) Application number: 07112192.5
(22) Date of filing: 10.07.2007
(51) Int. Cl.: G01N 33/68

(54) **Method for the prediction of the likelihood of a major adverse coronary event (MACE) in a patient with acute coronary syndromes (ACS)**

(71) Applicant: CellTrend GmbH, 14943 Luckenwalde (DE)
(72) Inventor: The designation of the inventor has not yet been filed
(74) Representative: Vossius & Partner

(57) **Abstract**

The present invention relates to a method for the prediction of the likelihood of a major adverse coronary event (MACE) in a patient with acute coronary syndromes (ACS), wherein the amount of vitronectin receptor gene product is determined in a s maple from a patient with acute coronary syndromes. Likewise the invention relates to the use of a kit for the prediction of the likelihood of a major adverse coronay event (MACE) in a patient with acute coronary syndromes (ACS) wherein the amount of vitronectin receptor gene products is determined in a sample from a patient with acute coronary syndromes and wherein the kit comprises a substance selected from the group of an antibody against sVNR and sVNR.

SEQUENCE LISTING

<110>  Celltrend GmbH

<120>  Method for the prediction of the likelihood of a major adverse
       coronary event (MACE) in a patient with acute coronary syndromes
       (ACS)

<130>  N1962 EP BLN

<160>  1

<170>  PatentIn version 3.3

<210>  1
<211>  38
<212>  PRT
<213>  Homo sapiens

<400>  1

Ser Arg Ala Arg Tyr Glu Met Ala Ser Asn Ser Arg Ala Arg Tyr Glu
1               5                   10                  15

Met Ala Ser Asn Pro Leu Tyr Arg Lys Pro Ile Ser Thr Pro Leu Tyr
            20                  25                  30

Arg Lys Pro Ile Ser Thr
        35

## Description

### Field of the invention

The invention is in field of chemistry and biology, more I particular diagnostics, more in particular *in vitro* diagnostics. The invention in particular is in the field of diagnosis of major adverse coronary events in patients with acute coronary syndromes.

### Background of the invention

Percutaneous coronary interventions (PCls) are associated with vessel wall injury at the treatment site. The local response is mediated by exposure of deep components of the arterial wall and stimulation of platelet adhesion and aggregation, thrombin generation, and fibrin deposition. The developing thrombus is highly thrombogenic and acts as a further stimulus to platelet deposition, thrombin generation, and fibrin formation. The clinical implications of intracoronary thrombosis after PCI are important because most short-term procedural complications are due to thrombosis; in addition, mural thrombus formation after PCI has been implicated in the pathogenesis of atherosclerosis and restenosis.

Activation of the platelet glycoprotein (GP) IIb/IIIa integrin initiates high-affinity binding of von Willebrand factor or fibrinogen, which is the final common pathway of platelet aggregation regardless of the initial thrombogenic stimulus. The addition of the mouse-human chimeric monoclonal antibody abciximab, which blocks GP IIb/IIIa and the related vβ3 receptor, to a regimen of aspirin and heparin decreases early postprocedural complications of PCl and improves the short-term clinical outcome. Platelet GP IIb/IIIa receptor blockade with abciximab inhibits platelet aggregation.

Recently, abciximab was also shown to decrease thrombin generation in a static system.

To date, there are few prediction markers available that will enable prediction of the likelihood of a major adverse coronary event in a patient with acute coronary syndromes.

### Summary of the invention

The inventors have found that determining the amount of vitronectin receptor gene product in a sample from a patient with acute coronary syndromes makes it possible to predict the likelihood of a major adverse coronary event (MACE) in a patient with acute coronary syndromes (ACS).

This finding is astonishing. Acute coronary syndromes (ACS) are characterized by increased platelet activation/aggregation. The vitronectin receptor (VNR), which promotes the binding of endothelial cells to platelets, is found in serum in a soluble form (sVNR). Abciximab reduces cardiac ischemic events associated with percutaneous coronary intervention (PCI) and binds equally well to the VNR and GP IIb/IIIa, as both share the β3 subunit. Assays were performed on baseline serum samples (n=233) from patients with similar interventions from the placebo-controlled, randomized trial of abciximab plus stenting (EPISTENT). Based on sVNR level, data were stratified into the lower 3 quartiles (n=178; < 49.7 ng/ml) versus the 4th quartile (n=55; ≥49.7 ng/ml). The primary endpoint was the occurrence of MACE (death, MI or urgent revascularization) at 30 days and 6 months. Univariate analysis demonstrated that patients with high sVNR (median 58.4 ng/ml, range: 49.9-101.8 ng/ml) were significantly more likely to have a MACE event compared with low sVNR (median 34.8 ng/ml, range: 7.5-49.6 ng/ml) at 30 days (18.2% vs 5.6%, p<0.05) and 6 months (20.0% vs 6.2%, p<0.01). A multivariate logistic regression model demonstrated that high sVNR significantly correlated with an increased risk of MACE (OR 3.94, 95% Cl 1.37-11.33) at 30 days while treatment with abciximab tended to reduce it (OR 0.37, 95% Cl 0.11-1.23) when other univariate predictors (MI history, troponin positive, congestive heart failure, diabetes, hypertension, and smoking status) were forced into the model. There was no treatment interaction with sVNR levels. If other predictors were allowed to drop out of the model, only sVNR (OR 3.23, 95%Cl 1.23-3.49) and history of MI (5.02, 95% Cl 1.41-17.9) remained. High sVNR levels were similarly predictive of events through 6 months (OR 3.36, 95%Cl 1.33-8.52).

The invention also relates to a kit for the prediction of the likelihood of a major adverse coronary event (MACE) in a patient with acute coronary syndromes (ACS), wherein the amount of vitronectin receptor gene product is determined in a sample from a patient with acute coronary syndromes and wherein the kit comprises a substance selected from the group of, an antibody against sVNR and sVNR.

### Definitions

(PCI) Percutaneous Coronary Interventions and Stents: The term Percutaneous Coronary Intervention (PCI) encompasses various procedures used to treat patients with coronary artery disease. PCI is also referred to as Percutaneous Transluminal Coronary Angioplasty (PTCA), Balloon Angioplasty, or just Angioplasty. PCI procedure: First performed in 1977, PCI or PTCA involves threading a slender balloon-tipped tube known as a catheter via the femoral artery in the groin (the brachial artery in the arm or radial artery in the wrist may also be used) to the narrowed or blocked coronary artery. The balloon is then inflated, compressing the plaque and dilating the narrowed artery, which enables blood to flow more easily.

Herein, a major adverse coronary event (MACE) is defined by death, myocardial infarction or target lesion (vessel) revascularization.

An unstable coronary syndrome is defined as postinfarction, refractory, or rest angina (Braunwald classes II or III, B or C).

High-risk lesions are defined as complex lesions or lesions of type B2 or C according to the American Heart Association/American College of Cardiology classification.

Complex lesions were defined as those with irregular borders or overhanging edges, with or without proximal or distal intracoronary filling defects (Ambrose criteria).

Herein, soluble Vitronectin Receptor is (sVNR or sVN-receptor) is the integrin alpha V beta 3, it consists of a 125 kDa alpha V subunit and a 105 kDa beta 3 subunit. It has been the focus of intensive research because of its major role in several distinct processes, particularly osteoclast mediated bone resorption, angiogenesis and pathological neovascularisation, and tumour metastasis.

The "sVNR" may be present in its natural cellular environment and can be used together with the material associated with the receptor in its natural state as well as in isolated form with respect to its primary, secondary and tertiary structures, the sVN-receptor is well known to those skilled in the art. Based on the weight of the whole receptor in the preparation to be used according to the invention, the isolated receptor should account for at least 0.5%, preferably at least 5% more preferably at least 25%, and in a particular preferred embodiment at least 50%. The receptor is preferably used in isolated form, *i.e.* essentially free of other proteins, lipids, carbohydrates or other substances naturally associated with the receptor. "Essentially free of" means that the receptor is at least 75%, preferably at least 85%, more preferably at least 95% and especially preferably at least 99% free of other proteins, lipids, carbohydrates or other substances naturally associated with the receptor.

In connection with the present invention, the naturally occurring receptor as well as all modifications, mutants, peptides or derivatives of the sVNR can be used. Similarly, a sVNR produced by means of recombinant techniques, which includes amino acid modifications, such as inversions, deletions, insertions, additions etc. can be used according to the invention provided that this part of the essential function of the sVNR is present, namely the capability of binding antibodies. The sVN-receptor being used may also comprise exceptional amino acids and/or modifications of such as alkylation, oxidation, thiol-modification, denaturation, oligomerization and the like. The receptor can also be synthesized by chemical means. According to the invention the sVNR particularly can be a protein and/or peptide or a fusion protein, which in addition to other proteins, peptides or fragments thereof, includes the sVNR as a whole or in part. Using conventional methods, peptides or polypeptides of the sVNR which have functionally analogs, analogous properties can be determined by those skilled in the art. For example such polypeptides or peptides have 50-60%, 70% or 80%, preferably 90%, more preferably 95%, and most preferably 98% homology to peptides identified as sVNR, and said homology can be determined, e.g. by means of Smith-Waterman homology search algorithm, using the MPFRCH Programme (Oxford Molecular), for example.

The term "peptide" of an sVNR used in the present invention, comprises also molecules differing from the original sequence by deletion(s), insertion(s), substitution(s) and/or other modifications well known in the prior art and/or comprising a fragment of the original amino acid molecule, the sVNR still exhibiting the properties mentioned above. Also included are allele variants and modifications. Methods of producing the above changes in the amino acid sequence are well known to those skilled in the art and have been described in the standard textbooks of molecular biology, e.g. Sam-brook *et al.* Supra. Those skilled in the art will also be able to determine whether an sVNR, thus, modified still has the properties mentioned above. Possible sVNR peptides used according to the invention can be, e.g. SRARYEMASNPLYRKPIST (SEQ ID NO.1) or fragments thereof. A fragment has at least 8 amino acids, preferably 9 amino acids, preferably 10 amino acids, preferably 11 amino acids, preferably 12 amino acids, preferably 13 amino acids, preferably 14 amino acids, preferably 15 amino acids, preferably 16 amino acids, preferably 17 amino acids, most preferably 18 amino acids amino acids.

In the present specification all of the above illustrated modifications of the sVNR will be referred to as "functionally analogous peptides or proteins".

"Sample" in the meaning of the invention can be all biological tissues and fluids such as blood, lymph, urine, cerebral fluid. The sample is collected from the patient and subjected to the diagnosis according to the invention.

The "anti-sVNR-antibody" in the meaning of the invention, which may be detected, in one embodiment of the invention, binds sVNR. The antibody can also be modified (e.g. oligomeric, reduced, oxidized and labeled antibodies). The term anti-sVNR-antibody antibody as used herein comprises both intact molecules and also anti-sVNR-antibody antibody fragments such as Fab, F(ab')₂ and Fv capable of binding specific epitope determinance of the sVNR. In these fragments the anti-sVNR-antibody(ies) capability of selectively binding its antigen or receptor is retained in part, the fragments being defined as follows:
(1) Fab, the fragment which contains a monovalent antigen-binding fragment of an antibody molecule, can be generated by cleavage of a whole antibody using the enzyme papaine, thereby obtaining an intact light chain and part of a heavy chain;
(2) the Fab fragment of an antibody molecule can be produced by treatment of a whole antibody with pepsin and subsequent reduction, thereby obtaining an intact light chain and part of a heavy chain, two Fab fragments per antibody molecule are obtained;
(3) F(ab')₂ the fragment of the antibody which can be obtained by treatment of a whole antibody with the enzyme pepsin without subsequent reduction, F(ab')₂ is a dimer comprised of two Fab fragments held together by two disulfate bonds;
(4) Fv defined as fragment modified by genetic engineering which includes the variable region of the light chain and the variable region of the heavy chain is expressed in the form of two chains; and
(5) single-chain antibody (SCA) defined as a molecule modified by genetic engineering, which includes the variable region of the light chain and the variable region of the heavy chain, linked by a suitable polypeptide linker to perform a genetically fused single-chain molecule.

The term "epitope" as used in the present invention represents any antigen determinant on the anti-sVNR-antibody. Epitope determinance normally consists of chemically active surface groups of molecules such as amino acids or sugar-side chains and normally has specific features of the free dimensional structure as well as specific chart properties.

The anti-sVNR-antibody binds specifically to the sVNR or in doing so shows specific immuno reactivity when the anti-sVNR-antibody assumes its function in a binding reaction in the presence of a heterogeneous population of sVNR or fragments thereof, thereby allowing a conclusion whether the sVNR or another biological structure is present. Under the present conditions of an immunoassay, the above-mentioned anti-sVNR-antibady will preferably bind to a specific portion of the sVNR, while no significant binding to other proteins present in the sample will take place.

"Patients" in the meaning of the invention are understood to be all persons, animals, plants or microorganisms, irrespective whether or not they exhibit pathological changes. In the meaning of the invention, any sample collected from cells, tissues, organs, organisms or the like can be a sample of a patient to be diagnosed. In a preferred embodiment the patient according to the invention is a human. In a further preferred embodiment of the invention the patient is a human.

An "immune reaction" in the meaning of the invention is a specific interaction between the sVNR or peptides or proteins of analogous function and anti sVNR antibodies. The immune reaction can be detected using various immunoassays.

"immunoassays" in the meaning of the invention are assays utilizing the specific interaction between the sVNR and peptides or proteins of analogous function and an anti-sVNR-antibody, in order to detect the presence or determine the concentration of sVNR. For example, a detection in quantification of the sVNR can be performed with the aid of said peptides or proteins of analogous function, e.g. by immuno precipitation or immuno blotting.

### Detailed description of the invention

The inventors have found that determining the amount of vitronectin receptor gene product in a sample from a patient with acute coronary syndromes makes it possible to predict the likelihood of a major adverse coronary event (MACE) in a patient with acute coronary syndromes (ACS). Thus, the invention relates to a method for the prediction of the likelihood of a major adverse coronary event (MACE) in a patient with acute coronary syndromes (ACS), wherein the amount of vitronectin receptor gene product is determined in a sample from a patient with acute coronary syndromes.

In a preferred embodiment the method is performed post percutaneous coronary intervention (PCI).

In one embodiment the invention relates to a method, wherein the amount of vitronectin receptor gene product is monitored over time. This may be at the beginning of testing and, e.g. in one weeks intervals thereafter, two weeks intervals thereafter, three weeks intervals thereafter, 4 weeks intervals thereafter, or at 30 days and then 6 months later. It is preferred that the method, *i.e.* the monitoring is performed PCI. In a preferred embodiment the monitoring is at 30 days and then 6 months later.

In one embodiment the invention relates to a method, wherein the amount of mRNA from the vitronectin receptor gene is determined.

In one embodiment the invention relates to a method, wherein the amount soluble vitronectin (sVS) receptor protein is measured.

In one embodiment the invention relates to a method, wherein the amount of sVNR is measured in an immunoassay.

For example, immunoassays in the meaning of the invention can be subdivided into the following steps:
(1) anti-sVNR-antibody/sVNR reaction,
(2) if required separation of the anti-sVNR-antibody/sVNR complex from other components of the reaction mixture especially from non-bound anti-sVNR-antibody and sVNR respectfully and
(3) measuring the amount.

As for the anti-sVNR-antibody/sVNR various configurations of passable, e.g. (a) precipitation of one reaction with an access of the other or (b) competition between known quantities of anti-sVNR-antibody or sVNR and the material to be investigated.

For example, an assay for sVNR can be performed by a) using access sVNR/peptides or proteins of analogous function or b) competition between a labeled anti-sVNR-antibody of known amount and non-labeled antibody of unknown amount for a defined quantity of sVNR or peptides of proteins of analogous function.

The sVNR can be immobilized on a solid support to allow separation of the anti-sVNR-antibody/sVNR complex. For example, the solid support material can be nitrocellulose, polyvinylchloride or polystyrene, e.g. the well of a microtiter plate. The immunoassay may take place in a microfluidic environment. To measure the anti-sVNR-antibody/sVNR interaction, it is possible to use labeled anti-sVNR-antibody, labeled sVNR or secondary reagents, for example. The sVNR can be labeled radioactively or with enzymes or with fluorescent compounds, for example. Irrespective of the label that is used, the response of the anti-sVNR-antibody/sVNR interaction can be enhanced by utilizing the affinity of the proteins avidine or streptavidine for biotin.

The immunoassays used according to the invention can be:
(1) immunoassays using radioactive label: (a) radioimmunoassay with competitive binding (RIA) and (b) immunoradiometric assay (IRMA);
(2) immunoassays using an enzyme label: (a) enzyme immunoassays (EIA) and (b) enzyme-linked immunosorbenassys (ELISA);
(3) immunoassays using a combination of radioisotope and enzyme labels (ultrasensitive enzyme radio immunoassay) (USERIA).

Further, the immunoassay according to the invention may be a fluorescent immunoassay, a chemiluminescent assay, an agglutination assay, a nephelometric assay, a turbidimetric assay, a Western Blot, a competitive immunoassay, a non-competitive immunoassay, a homogenous immunoassay, a heterogenous immunoassay, a reporter-assay, e.g. a luciferase assay.

In a preferred embodiment of the invention the immunoassay is an ELISA.

In one embodiment of the invention the invention relates to a method, wherein an elevated level of sVNR is indicative of a higher chance of MACE.

In one embodiment of the invention the invention relates to a method, wherein said elevated level is above 49,6 ng/ml when measured in serum.

In one embodiment of the invention the invention relates to a method, wherein a high level of sVNR is indicative of a significantly higher likelihood of MACE.

In one embodiment of the invention the invention relates to a method, wherein said high level of sVNR is over 49,9 ng/ml.

In one embodiment of the invention the invention relates to a method, wherein the ELISA is a competitive ELISA.

In one embodiment of the invention the invention relates to a method, wherein the competitive ELISA comprises an sVNR which is solid phase bound.

The immunological test kit according to the invention comprises the sVNR or a functional analog thereof or peptides or proteins of analogous function per se. The test kit of the invention comprises at least one complete sVNR or functionally analogous peptides or proteins of said receptor, optionally bound to a solid phase. Furthermore, the test kit may also comprise buffers, specific conjugate together with an enzyme, wash solution, substrate solution to detect the immune reaction and/or a quenching solution. Using these substances a person skilled in the art will be able to perform, e.g. an ELISA to detect the anti-sVNR antibodies. The buffers, specific conjugate plus enzyme, wash solution, substrate solution to detect immune reaction and quenching solution are well known to those skilled in the art. For example, it would be sufficient to have the test comprise a freeze-dried sVNR or peptides or proteins of sVNR analogous function and to add the buffers and other solutions immediately prior to testing the biological material. However, it is also possible to provide the test kit with the sVNR or its functionally analogous peptides of proteins bound to a solid phase. To detect the anti-sVNR antibodies the specific conjugate, wash solution, substrate solution and quenching solution, which can be components of the test kit, have to be added according to a mode well known to those skilled in the art.

In another advantageous embodiment of the invention, it is envisioned that the test kit is a test strip comprising the sVNR or its functionally analogous peptides or proteins immobilized on a solid phase. For example, the test strip can be immersed in serum or other patient samples and incubated. Using a specific biochemical reaction on the test strip after formation of the sVNR /anti-sVNR antibody complex, a specific colour reaction can be triggered by means of which the anti-sVNR antibody can be detected.

The test system of the invention permits quantification of the sVNR antibodies directly in a sample, e.g. in plasma of patients. The detection method according to the invention is time saving and cost effected. Large amounts of the samples can be tested and, owing to the low amount of the equipment required, routine laboratories can be used.

### Examples

The VNR-ELISA is a solid phase ELISA based on a kompetitive method that utilizes a vitronectin receptor and polyclonal antibodies against vitronectin receptor. The vitronectin receptor is bound to a microtiter plate to create the solid phase. Nonspecific biding is blocked by a blocking buffer. Then samples, standards and VNR antibodies are simultaneiously added to the wells of plates. During the incubation, the VNR antibodies bound to the solid pahse VNR and to the sVNR. After a washing step a second POD-labeled anti-VNR-antibody is added. The reaction between POD and substrate results in colour development. The colour intensity is inverse proportional to the amount of VNR present in samples and standards. The amount of VNR can be quantitated by measuring absorbance using an ELISA plate reader.

### Specimen collection and handling:

Venous blood samples are collected aseptically. Serum is suitable for use in the assay. EDTA, or citrated plasma, cultured cell extracts or cell culture supernatant can also be used. Remove the serum or plasma from the clot or red cells. Freeze-thaw cycles should be avoided.

### Figure captions

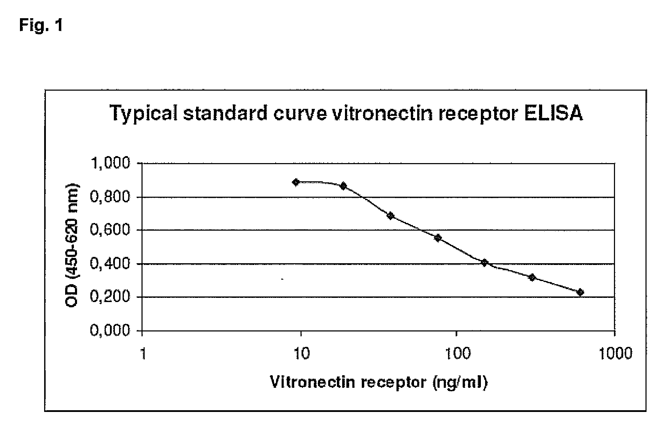

Fig. 1 shows a typical standard curve vitronectin receptor ELISA. The range of standard curve is 18 - 600 ng/ml. The test specimen type is human serum or plasma, cell culture supernatants, cell extracts.

## Claims

1. Method for the prediction of the likelihood of a major adverse coronary event (MACE) in a patient with acute coronary syndromes (ACS), wherein the amount of vitronectin receptor gene product is determined in a sample from a patient with acute coronary syndromes.

2. Method according to claim 1, wherein the amount of vitronectin receptor gene product is monitored over time.

3. Method according to claims 1 to 2, wherein the amount of mRNA from the vitronectin receptor gene is determined.

4. Method according to claim 1 or 2, wherein the amount soluble vitronectin (sVNR) receptor protein is measured.

5. Method according to claim 3, wherein the amount of sVNR is measured in an immunoassay.

6. Method according to claim 4 to 5, wherein an elevated level of sVNR is indicative of a higher chance of MACE.

7. Method according to claims 4 to 6, wherein said elevated level is above 49,6 ng/ml when measured in serum.

8. Method according to claims 4 to 6, wherein a high level of sVNR is indicative of a significantly higher likelihood of MACE.

9. Method according to claim 8, wherein said high level of sVNR is over 49,9 ng/ml.

10. Method according to claims 5 to 9 wherein, the immunoassay is selected from the group of immunoprecipitation, enzyme immunoassay (EIA)), radioimmunoassay (RIA) or fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay.

11. Method according to claim 10, wherein the immunoassay is an ELISA.

12. Method according to claim 11, wherein the ELISA is a competitive ELISA.

13. Method according to claim 12, wherein the competitive ELISA comprises an sVNR which is solid phase bound.

14. Use of a kit for the prediction of the likelihood of a major adverse coronary event (MACE) in a patient with acute coronary syndromes (ACS), wherein the amount of vitronectin receptor gene product is determined in a sample from a patient with acute coronary syndromes and wherein the kit comprises a substance selected from the group of, an antibody against sVNR and sVNR.
